# FASCICULE DE BREVET EUROPEEN

(11) **EP 3 684 441 B1**
(45) Date de publication et mention de la délivrance du brevet: **06.11.2024**
(21) Numéro de dépôt: 18783368.6
(22) Date de dépôt: 19.09.2018
(51) Int. Cl.: A61M 60/17, A61M 60/221, A61M 60/232, A61M 60/237, A61M 60/422, A61M 60/806, A61M 60/812, F04D 13/06, F04D 29/18

(54) **POMPE CARDIAQUE EQUIPEE D'UNE TURBINE A PALES INTERNES**
HERZPUMPE MIT EINER TURBINE MIT INTERNEN SCHAUFELN
CARDIAC PUMP HAVING A TURBINE WITH INTERNAL BLADES

(30) Priorité: 21.09.2017 FR 1758740
(43) Date de publication de la demande: 29.07.2020
(73) Titulaire: Fineheart, 33600 Pessac (FR)
(72) Inventeur: HADDADI, Mohammad, 33700 Merignac (FR); GARRIGUE, Stéphane, 33130 Begles (FR); MASCARELL, Arnaud, 37250 Montbazon (FR)
(74) Mandataire: IPAZ
(86) Numéro de dépôt international: PCT/EP2018/075362
(87) Numéro de publication internationale: WO 2019/057779

(56) Documents cités:
- US-A- 5 368 438
- US-A1- 2001 009 645
- US-A1- 2013 209 292
- US-A1- 2016 279 311
- US-A1- 2016 375 187

## Description

La présente invention se rapporte à une pompe pour la circulation d'un fluide. Elle trouve une application particulièrement intéressante dans le domaine de la régulation du débit sanguin. En effet, une telle pompe peut être implantée dans le ventricule d'un coeur de façon à augmenter le débit sanguin et l'adapter au besoin du patient. Bien évidemment, une attention toute particulière doit être portée aux caractéristiques de la pompe. Cette pompe doit être biocompatible et fournir un minimum de confort au patient.

La pompe selon l'invention peut également être utilisée hors ventricule, en position verticale ou horizontale, de façon connectée ou non à des capteurs.

On connaît le document WO2017032510 décrivant une pompe dotée d'une turbine en forme d'ogive. Cette turbine est équipée de plusieurs pales réalisées sur sa surface. Bien qu'une telle pompe fonctionne avec de bonnes performances, elle présente toutefois quelques inconvénients comme le poids et le niveau de contrainte sur des cellules sanguines. US 2016/375187 A1 décrit des pompes à sang utilisant un rotor creux.

La présente invention a pour objet une nouvelle pompe réduisant la contrainte sur les cellules sanguine.

Un autre objet de l'invention et de limiter les frottements à l'intérieur de la pompe.

L'invention a encore pour objet de réduire la consommation énergétique du moteur de la pompe.

On atteint au moins l'un des objectifs avec une pompe conçue pour la circulation d'un selon la revendication 1.

La pompe selon l'invention comprend une turbine de nouveau genre qui est creuse et comporte des pales internes, c'est-à-dire dirigées vers l'axe centrale de rotation. En effet, contrairement aux turbines ou rotors connus qui possèdent généralement un corps principal avec des pales disposées sur la surface externe, la présente invention propose une turbine creuse avec des pales réalisées dans sa surface interne. Le fluide est amené à traverser la turbine, en son intérieur, directement, selon un flux linéaire, avec un minimum de frottement.

Une telle architecture permet de diminuer le poids de la turbine par rapport à une turbine pleine.

Avec une telle pompe implantée dans un ventricule du coeur d'un patient, le confort du patient est grandement amélioré. Par ailleurs, la consommation électrique est aussi améliorer du fait du faible poids. Il s'ensuit un dimensionnement de la source énergétique plus compact et/ou une durée de vie améliorée pour une telle pompe.

Comme le fluide passe à l'intérieur de la turbine, on diminue la résistance au flux.

On réduit considérablement la contrainte, c'est-à-dire les forces de cisaillement (« shear stress ») sur des cellules sanguines lorsque le fluide est le sang. Le sang est par conséquent moins dégradé.

Avantageusement, le carter et la turbine peuvent constituer une partie d'un moteur sans balais, ou moteur « brushless », le carter ayant la fonction de stator et la turbine ayant la fonction de rotor. On constitue ainsi une machine synchrone à aimants permanents disposés dans le rotor, alors que des enroulements statoriques (bobinages) seraient réalisés dans le carter utilisé comme stator. Dans ce mode de réalisation, l'électronique de commande est prévu est disposé de façon à agir sur les bobinages. Ce mode de réalisation consiste à avoir un moteur intégré.

La pompe selon l'invention comprend une tige disposée dans l'axe centrale de la turbine de façon à réduire les refoulements de fluide. Cette tige est disposée au centre du carter.

La tige est fixe. Même si cela ne constitue pas un mode de réalisation préféré, on peut toutefois imaginer une tige en rotation, solidaire ou pas de la turbine, fixée ou pas à la chambre d'admission.

Par exemple, cette tige peut être fixée à ladite au moins une pale ou au corps de la turbine ou par une extrémité à une chambre d'admission qui est solidaire du carter. Lorsque la tige est fixée à la chambre d'admission, la turbine peut pivoter autour d'elle.

La fonction de cette tige est de contribuer à la suppression d'éventuels reflux de sang et à l'empêchement de la création de vortex.

Selon un autre mode de réalisation de l'invention, la pompe peut comprendre un moteur extérieur à la turbine et entraînant un arbre de transmission, ce dernier étant disposé dans l'axe de rotation de la turbine et solidement connecté par au moins une arrête radiale à la turbine. Contrairement au cas précédent où le moteur était finalement intégré, dans le présent mode de réalisation, le moteur est à l'extérieur de la turbine et comporte un arbre qui entraîne la turbine en rotation. L'arbre est solidement fixé à la turbine par des arêtes qui ont un profil permettant le passage aisé du fluide.

De préférence, l'arbre de transmission peut être à la fois connectée à l'extrémité amont et à l'extrémité avale de la pale.

Selon une caractéristique de l'invention, la pompe comprend en outre:
- un inducteur doté d'aubes de guidage pour rendre l'écoulement du fluide linéaire ; cet inducteur étant disposé en amont de la turbine par rapport au sens de circulation du fluide ;
- un diffuseur doté d'aubes de diffusion pour rendre l'écoulement de fluide linéaire et augmenter la pression du fluide; ce diffuseur étant disposé en aval de la turbine de façon à évacuer le fluide depuis la turbine vers l'extérieur en transformant l'énergie cinétique créée par la turbine en énergie potentielle ; et
- un redresseur doté d'aubes redresseurs et d'un orifice en sortie de diamètre inférieur au diamètre d'entrée du redresseur, les aubes redresseurs dirigeant le fluide, en provenance du diffuseur, vers l'orifice de façon à augmenter la vitesse et donner au fluide un profil prédéterminé en sortie de l'orifice.

L'écoulement de fluide linéaire telle que défini ici, l'est par opposition à un écoulement tourbillonnant. Cet écoulement linéaire peut être laminaire.

Le redresseur (« straightener » en langue anglaise) selon l'invention permet de créer un flux en concentrant le fluide de façon à obtenir des valeurs de vitesses élevées en sortie de la pompe. De façon générale, le système vasculaire d'un coeur présente des résistances vasculaires assez élevées. Une pompe efficace est une pompe capable de propulser le sang dans les valves avec une pression suffisante pour vaincre ces résistances vasculaires. La pression en sortie de la pompe est primordiale comparativement à la vitesse de sortie qui avec la pompe selon l'invention peut atteindre la vitesse maximale de 3 m/s.

En d'autres termes, le redresseur permet de canaliser le fluide, de créer un profil permettant d'avoir une vitesse maximale en un point, i.e. aligné avec la valve aortique, de manière à expulser le flux de façon laminaire. Ceci permet d'éviter la création de tourbillons en sortie de la pompe. Le diamètre de l'orifice de sortie est plus petit que le diamètre interne du carter. Son petit diamètre de sortie, par exemple un demi ou un tiers du diamètre interne du carter, permet de régler les paramètres de pression, entre 80 et 200 mmHg, et de vitesse, entre 1 et 3 m/s, tout en évitant la création d'un courant à vitesse négative (en contresens par manque de pression et d'homogénéité du flux en sortie).

L'ensemble diffuseur et redresseur selon l'invention permet donc de rendre la pompe efficace. Le redresseur diffuse le fluide directement vers la sortie dans le milieu ambiant en créant un flux. Ce milieu ambiant peut avantageusement être ledit fluide, qui est de préférence du sang.

L'inducteur selon l'invention évite le phénomène de cavitation, c'est-à-dire la création de bulle dans le fluide.

Il est important de noter que cette pompe est parfaitement adaptée pour un fonctionnement selon une disposition verticale, ou légèrement penchée c'est-à-dire inclinée entre 0 et 5 degrés par rapport à l'axe verticale. La pompe selon l'invention peut également fonctionner couchée comme la plupart des pompes de l'art antérieur.

La pompe selon l'invention peut en outre comprendre une chambre d'admission pourvue d'ouvertures latérales pour que le fluide puisse pénétrer radialement puis s'engager dans l'axe vers l'inducteur. Cette chambre d'admission peut être de forme cylindrique comportant sur sa partie supérieure en aval desdites ouvertures, un réceptacle pour accueillir l'inducteur. La chambre d'admission et l'inducteur peuvent constituer deux pièces destinées à être fermement solidaires l'une de l'autre ou bien être conçus comme une seule et unique pièce. Le carter peut également être associé avec eux de façon à constituer une pièce unique.

Selon une caractéristique avantageuse de l'invention, la pale est de type hélicoïdal. Une telle pale a pour fonction d'aspirer le fluide et de le faire traverser l'intérieur de la turbine.

Cette pale peut être plus ou moins marquée, c'est-à-dire qu'elle peut être une ou des pièces rapportées par rapport à la turbine ou bien des excroissances, des sillons ou des ondulations faisant partie intégrale de la turbine, réalisées sur sa paroi interne.

La pale hélicoïdale est comme un serpentin sur la paroi interne de la turbine de telle sorte que l'angle d'attaque de la pale hélicoïdale diminue au fur et à mesure que l'on se déplace de l'extrémité amont de la turbine vers l'extrémité en aval; l'angle d'attaque étant défini comme l'angle entre l'axe de rotation de la turbine et un vecteur tangent à la surface externe de la pale hélicoïdale.

Selon une autre caractéristique avantageuse de l'invention, la pompe comprend plusieurs pales réparties sur la paroi interne dudit corps. Ces pales sont disposées pour appliquer un minimum de contrainte au fluide et optimiser l'aspiration.

Selon un mode de réalisation de l'invention, la pale est constituée de plusieurs spires à pas d'enroulement croissant et tendant vers l'infini.

Avantageusement, la partie centrale creuse de la turbine peut être de forme cylindrique. C'est-à-dire que la paroi interne de la turbine peut présenter le profil d'un cylindre droit, à section circulaire par exemple, et/ou l'épaisseur radiale fixe ou variable de la pale est telle que l'espace vide (rempli d'air ou de fluide) présente le profil d'un cylindre droit, à section circulaire par exemple.

Selon un mode de réalisation de l'invention, la partie centrale creuse peut être de forme évasée, l'extrémité la plus ouverte étant disposée en aval selon le sens de circulation du fluide.

L'orifice de sortie de la turbine est plus grand que l'orifice d'entrée.

Selon un autre mode de réalisation de l'invention, la partie centrale creuse de la turbine est de forme oblongue, l'extrémité la plus ouverte étant disposée en aval selon le sens de circulation du fluide.

De préférence, pour une rotation efficace, le corps de la turbine peut présenter une forme extérieure de type cylindre droit à section circulaire.

Selon un mode de réalisation, l'épaisseur radiale de la pale peut être fixe ou variable sur toute la longueur entre l'extrémité amont et l'extrémité aval de cette pale, c'est-à-dire sur toute la longueur de la pale. Si elle reste constante, les pales délimitent alors une zone qui présente une forme sensiblement identique à la forme de la partie creuse.

Selon une caractéristique avantageuse de l'invention, la partie centrale creuse et ladite au moins une pale présentent un profil de type centrifuge du côté amont, de type mixte dans la partie centrale, et de type axial du côté aval.

La pompe selon l'invention intègre les caractéristiques à la fois :
- d'une pompe de type centrifuge, c'est-à-dire une accélération radiale pour la partie du fluide prise par les pales et arrivant axialement, pour ce faire la partie basse des pales, notamment hélicoïdales, peuvent présenter un angle prononcé, de l'ordre de l'ordre de 45 degrés, par exemple entre 40 et 50 degrés, par rapport à l'axe de rotation de la turbine,
- d'une pompe de type mixte, c'est-à-dire une pente ou une courbure moins prononcée des spires des pales hélicoïdales par exemple, et
- d'une pompe de type axial, le fluide étant conduit selon une direction parallèle à l'axe de rotation de la turbine.

Cette configuration permet de limiter les forces de cisaillement (« shear stress » en langue anglaise) qui peuvent engendrer une hémolyse, c'est-à-dire une destruction des globules rouges. Même avec un fort débit, lorsque les globules rouges sont détruits, l'oxygène ne parvient pas jusqu'aux cellules.

Les forces de cisaillement sont créées par des effets de tourbillonnement de sang en entrée et en sortie de pompe. La pompe selon l'invention évite les tourbillons par un appel de sang en utilisant une structure de type centrifuge et un refoulement axial.

Selon une caractéristique avantageuse de l'invention, une première zone de ladite au moins une pale est dimensionnée pour que le fluide atteigne une vitesse spécifique comprise entre 0 et 1.2 ; cette première zone étant du côté amont de la pale.

Par ailleurs, une deuxième zone de ladite au moins une pale est dimensionnée pour que le fluide atteigne une vitesse spécifique comprise entre 1 et 2.2 ; cette deuxième zone étant au niveau du milieu de la pale.

Enfin, une troisième zone de ladite au moins une pale est dimensionnée pour que le fluide puisse atteindre une vitesse spécifique supérieure à 2.2 ; cette deuxième zone étant du côté aval de la pale. Il s'agit de valeur de vitesse spécifique permettant de classifier les structures centrifuge, mixte ou axial respectivement.

D'autres avantages et caractéristiques de l'invention apparaîtront à l'examen de la description détaillée d'un mode de mise en oeuvre nullement limitatif, et des dessins annexés, sur lesquels :
La figure 1 est une vue schématique d'une turbine à pales intérieures selon l'invention,
Les figures 2a, 2b et 2c sont des schémas illustrant la disposition des pales hélicoïdales à l'intérieur de la turbine selon l'invention,
Les figures 3a, 3b et 3c sont des vues en coupe schématiques illustrant un moteur « brushless » à courant continu sous différentes configurations de la partie centrale creuse de la turbine selon l'invention,
La figure 3d est une vue en coupe schématique illustrant un moteur de type de ceux décrits sur les figures 3a à 3c avec une tige disposée au centre de la turbine,
La figure 3e est une vue schématique d'une chambre d'admission portant la tige de la figure 3d,
La figure 4 est une vue schématique par transparence de l'intérieure d'une pompe intégrant une turbine selon l'invention,
La figure 5 est une vue éclatée de la pompe intégrant une turbine selon l'invention,
La figure 6 est une vue en coupe de la pompe des figures 4 et 5,
La figure 7 est une vue schématique en coupe longitudinale d'une pompe selon l'invention,
La figure 8 est une vue en perspective d'une chambre d'admission selon l'invention,
La figure 9 est une vue en perspective d'un inducteur à insérer dans la chambre d'admission selon l'invention,
La figure 10 illustre différentes vues en coupe et en perspective d'un carter destin à contenir la turbine selon l'invention,
La figure 11 est une vue en perspective d'un diffuseur selon l'invention,
La figure 12 est une vue en perspective d'un redresseur selon l'invention,
La figure 13 est une vue en perspective d'une turbine fixée à un arbre de transmission d'un moteur selon l'invention,
La figure 14 est une vue éclatée de la pompe intégrant la turbine de la figure 13 selon l'invention,
La figure 15 est une vue schématique illustrant une fixation entre un arbre de transmission et la turbine de la figure 13 selon l'invention,
La figure 16 est une vue schématique en coupe laissant apparaître l'intérieur d'une pompe intégrant la turbine de la figure 13 selon l'invention,
La figure 17 est une vue schématique d'une pompe selon l'invention comprenant un moteur dont l'arbre de transmission est connecté à la turbine de la figure 13 selon l'invention,
Les figures 18 et 19 sont des vues schématiques d'un inducteur selon l'invention, et
Les figures 20 et 21 sont des vues schématiques d'un redresseur selon l'invention.

Les modes de réalisation qui seront décrits dans la suite ne sont nullement limitatifs ; on pourra notamment mettre en oeuvre des variantes de l'invention ne comprenant qu'une sélection de caractéristiques décrites par la suite isolées des autres caractéristiques décrites, si cette sélection de caractéristiques est suffisante pour conférer un avantage technique ou pour différencier l'invention par rapport à l'état de la technique antérieur. Cette sélection comprend au moins une caractéristique de préférence fonctionnelle sans détails structurels, ou avec seulement une partie des détails structurels si cette partie uniquement est suffisante pour conférer un avantage technique ou pour différencier l'invention par rapport à l'état de la technique antérieur.

En particulier toutes les variantes et tous les modes de réalisation décrits sont prévus pour être combinés entre eux dans toutes les combinaison où rien ne s'y oppose sur le plan technique.

Sur les figures, les éléments communs à plusieurs figures conservent la même référence.

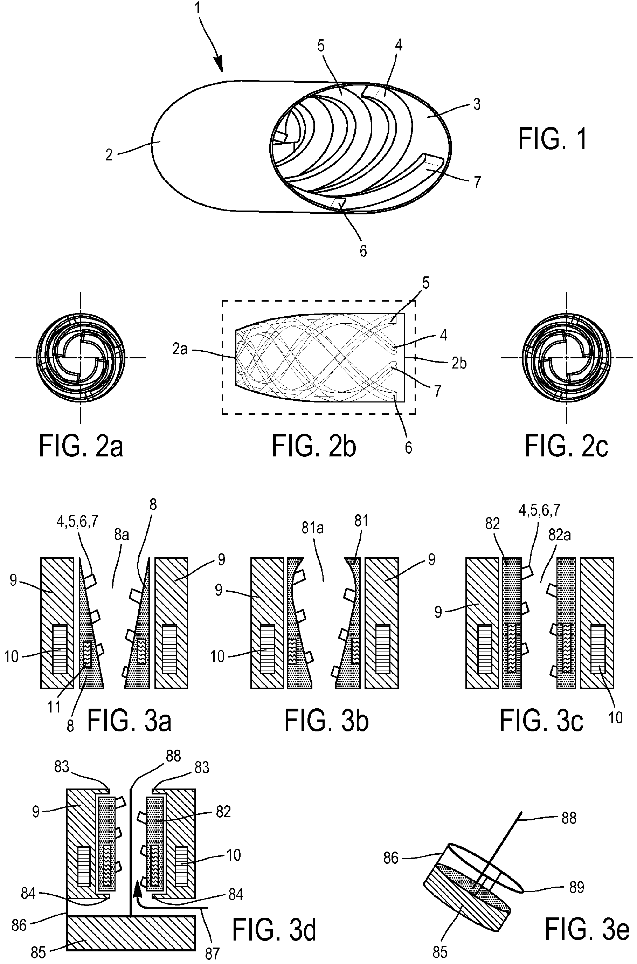

Sur la figure 1 on distingue une turbine 1 selon l'invention. Il s'agit, dans l'exemple décrit de façon non limitative, d'un corps 2 en forme de cylindre creux longiligne dont la paroi interne 3 comporte plusieurs pales 4, 5 6 et 7. Une telle turbine peut avantageusement être utilisée dans une pompe plongée dans un fluide.

Les pales ont pour fonction de faire transiter le fluide à travers la turbine. L'orientation et le dimensionnement des pales sont prévus pour que le fluide soit aspiré puis propulsé après avoir traversé la turbine qui serait en rotation.

Contrairement à des systèmes de l'art antérieur où le fluide circule à l'extérieur de la turbine, la présente invention propose une turbine creuse faisant circuler le fluide à l'intérieur.

La figure 2b illustre les quatre pales hélicoïdales 4 à 7, qui partent d'une extrémité de la turbine, s'inscrivent dans des lignes hélicoïdales sans jamais se croiser, et arrivent à l'autre extrémité.

Dans l'exemple de la figure 2b, la partie centrale creuse du corps 2 présente une forme évasée concave. L'extrémité amont 2a présente une ouverture plus réduite que l'extrémité aval. La figure 2a est une vue de face de l'extrémité 2a alors que la figure 2c est une vue de face de l'extrémité 2c. Les pales hélicoïdales suivent la forme de la paroi interne 3.

La partie centrale creuse peut avoir d'autres formes comme celles représentées sur les figures 3a à 3c de façon non limitative.

Sur la figure 3a, une turbine 8 est représentée en coupe. Il s'agit d'un cylindre ayant une partie centrale creuse 8a de forme évasée telle un entonnoir. Une pale 4 est schématiquement illustrée. Bien entendu plusieurs pales 4, 5, 6 ou 7 peuvent être disposées. Cette turbine 8 est conçue pour être en rotation par rapport à un carter 9 l'entourant. Idéalement le carter 9 est un stator comportant des enroulements magnétiques 10. Ces derniers peuvent coopérer magnétiquement avec des aimants permanents 11 disposés à l'intérieur ou sur la surface externe de la turbine. Des moyens électroniques (non représentés) sont prévus pour commander les enroulements 10 de façon à mettre en action la turbine. L'ensemble constitue un moteur de type « brushless ». L'entrefer entre la turbine et le carter est droit, il s'inscrit dans un cylindre droit, mais il peut être d'une autre forme non droite, évasée ou non.

Sur la figure 3b, la partie centrale creuse 81a présente une forme oblongue comme sur la figure 2b mais avec une extrémité en aval qui se referme légèrement.

Sur la figure 3c, la partie centrale creuse 82a présente la forme d'un cylindre droit.

Sur les trois figures 3a, 3b et 3c on retrouve le même carter 9 ainsi que les mêmes éléments magnétiques permettant de constituer un moteur de type « brushless ».

Il a été constaté que la disposition d'une tige à l'intérieur de la turbine permet d'améliorer l'écoulement du sang lorsque la pompe est en fonctionnement, c'est-à-dire la turbine en rotation par rapport au carter. La figure 3d illustre un exemple de réalisation d'une pompe dans laquelle une tige 88, droite, est disposée dans l'axe de la turbine. Dans l'exemple décrit sur la figure 3d, la pompe comporte une chambre d'admission 85, 86 que l'on voit sur la figure 3e. Cette chambre d'admission comprend une base 85 portant des entretoises 86 reliées à une couronne 89 de fixation au carter 9. Les passages entre la couronne 89 et la base 85 permettent l'entrée 87 du fluide ou du sang vers l'intérieur de la turbine le long de la tige 88, sous l'action des pales 4, 5, 6, 7. Cette tige 88 peut avoir une hauteur telle que son extrémité libre (supérieure sur la figure 3d) se trouve à l'intérieure de la turbine, au-delà de la turbine, affleurant l'extrémité supérieure de la turbine ou du carter.

La tige 88 est fixée par une extrémité à la base 85. D'autres modes de disposition de la tige peuvent être envisagées, comme par exemple reliée uniquement à la turbine et non plus à la base 85. En effet, la tige 88 peut être fixée au corps 82 de la turbine ou à l'une des pales 4, 5, 6, 7 par exemple par le haut, à l'extrémité supérieure (par rapport à la représentation de la figure 3d). Dans ce cas, l'extrémité inférieure de la tige 88 peut être de préférence libre ou bien fixée à une pale, au corps de la turbine ou à la chambre d'admission.

En complément notamment de ce qui précède et de façon compatible avec d'autres modes de réalisation, la turbine peut être contenue dans le carter au moyen de rebords 83 et/ou 84 réalisés par exemple aux extrémités des du carter et s'étendant vers l'intérieur. Des mécanismes à roulement peuvent être prévus entre la turbine et les rebords. D'autres moyens peuvent être prévus pour maintenir la turbine en rotation dans le carter sans contact. On peut notamment envisager un mécanisme par sustentation, par fluide, par aimantation,..., avec ou sans les rebords 83 et/ou 84.

Les figures 1 à 3 décrivent notamment une turbine et un carter qui, associés à des composants d'admission et de sortie de fluide et à des composants électroniques, permettent de constituer une pompe.

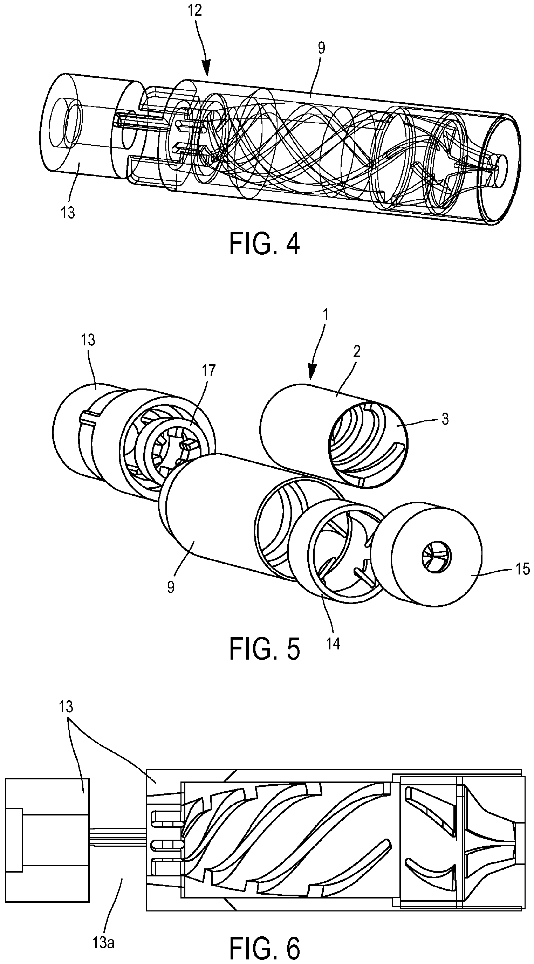

On va maintenant décrire en référence aux figures 4 à 6, une pompe selon l'invention intégrant une turbine telle que décrite ci-dessus.

Sur la figure 4, on distingue une vue globale en transparence d'une pompe 12. Cette pompe est sous la forme globale d'un cylindre à section circulaire, destinée à aspirer un fluide tel que le sang et le refouler de façon à favoriser la circulation sanguine. Une telle pompe est destinée à être installée dans un corps notamment pour assistance ventriculaire. Sa longueur est d'environ 61.8mm, le diamètre du carter 9 est d'environ 17 - 20 mm, alors que la partie basse 13 présente un diamètre d'environ 15 - 20 mm.

La pompe selon l'invention peut avantageusement, mais pas uniquement, être utilisée dans un positionnement vertical, c'est-à-dire le carter 9 à la verticale et au-dessus de la partie basse 13.

Selon l'invention, la chambre d'admission 13 a pour fonction de faire entrer le fluide, notamment du sang, par les ouïes ou ouvertures 13a sous l'action d'une aspiration provenant de l'intérieur du carter 9. Le fluide est ensuite refoulé par une ouverture à l'extrémité supérieure du carter.

Les étages supérieurs de la chambre d'admission 13 comprennent la turbine 1 destinée à se mouvoir en rotation à l'intérieur du carter 9, et des éléments de sortie tels un diffuseur 14 et un redresseur 15. La turbine 1 visible sur la vue éclatée de la figure 5 présente une partie centrale creuse de forme oblongue, ovale ou une ogive allongée ou étirée sur une extrémité. La partie centrale creuse délimitée par la paroi interne 3 présente un diamètre de la section circulaire (coupe radiale) qui croît depuis sa partie basse jusque vers la partie supérieure.

Sur la figure 5, la vue éclatée permet de comprendre que les deux composants globalement visibles de l'extérieur sont le carter 9 ayant une partie inférieure réduite destinée à s'insérer sur la partie supérieure de la chambre d'admission 13. La turbine 1 est insérée à l'intérieure du carter 9, dans la partie centrale. Le diffuseur 14 et le redresseur 15 s'emboîte dans le carter 9 par l'extrémité supérieure.

Sur la figure 6, on distingue clairement les pales hélicoïdales réalisées sur la paroi interne 3.

Les différentes pièces de la pompe peuvent être conçues par moulage, impression 3D, usinage ou autres. Avantageusement, on réalise entre trois et cinq pales.

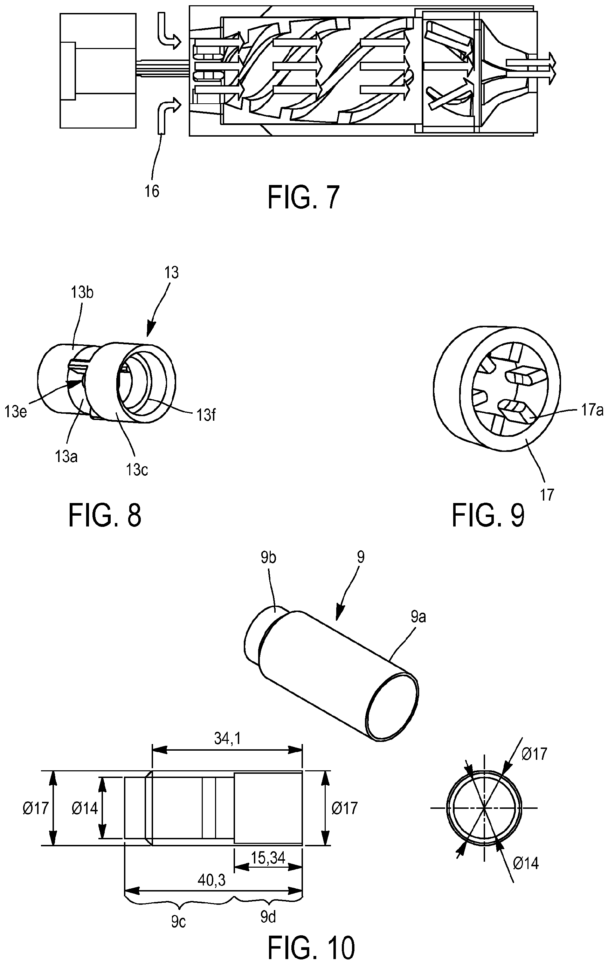

Sur la figure 7, des flèches16 indiquent le parcours du fluide à l'intérieur de la pompe. Le fluide pénètre par les ouvertures 13a de la chambre d'admission puis est accéléré axialement dans la turbine sous l'action rotative des pales. Chaque pale hélicoïdale est un serpentin d'épaisseur constante ou variable sur toute la longueur de sorte que la forme de l'espace intérieure est conforme ou pas à la forme de la paroi interne 3. Cette forme intérieure est oblongue ou de type ogive.

La turbine axiale selon l'invention est prévue pour un fonctionnement continu.

La circulation du fluide se fait selon un flux linéaire qui occupe tout l'espace central de la turbine. Le débit est ainsi important et le fluide, lorsqu'il s'agit notamment du sang, subit le moins de choc possible, donc le moins de contrainte possible sur les cellules.

Sur la figure 8 on voit plus en détail la chambre d'admission 13 constituée d'une partie basse 13b, d'une partie supérieure 13c, les deux parties étant reliées par des guides radiaux 13d délimitant les ouvertures 13a vers l'intérieur de la chambre d'admission.

La partie basse 13b est un cylindre de section circulaire, de paroi épaisse de telle sorte que la partie centrale est un tunnel 13e. Dans l'exemple de la figure 8, le diamètre du tunnel est de 6 mm.

Les guides radiaux 13d sont trois plaques inscrites dans des plans qui se coupent sur l'axe de la chambre d'admission. La face extérieure de chaque plaque 13d affleure la surface extérieure latérale de la partie supérieure 13c. La zone centrale de la partie supérieure contenant l'axe de la chambre d'admission est vide pour le passage du fluide. Cette zone centrale constitue un tunnel de diamètre supérieur au diamètre du tunnel 13e.

La partie supérieure 13c est sous la forme d'un cylindre présentant deux épaisseurs différentes, une première épaisseur du côté en amont, c'est-à-dire du côté en contact avec les guides radiaux 13d, et une seconde épaisseur, inférieure à la première, du côté aval. Entre les deux épaisseurs se trouve une marche 13f. Avec une telle disposition, un inducteur 17 tel que décrit sur la figure 9 peut être inséré et fixé à l'intérieur de la chambre d'admission 13 dans la partie 13c de grande épaisseur. Lors de l'insertion, cet inducteur 17 peut venir se poser sur des extrémités des guides 13d. Les dimensions de l'inducteur 17 sont telles qu'une fois inséré, sa partie supérieure affleure la marche 13f. On peut envisager d'autres modes de réalisation telles que par exemple une pièce unique constituée des éléments 13c et 17, ou alors 13 et 17. L'inducteur 17 est un cylindre creux comportant des guides radiaux 17a, par exemple au nombre de quatre jusqu'à six, sur toute la hauteur du cylindre et s'inscrivant dans des plans radiaux concourants au centre du cylindre. L'inducteur 17 sert de guide d'entrée du fluide. Il permet de limiter la cavitation dans les étages supérieurs. Les guides 17a produisent un écoulement laminaire de sorte que le caractère turbulent du fluide est considérablement réduit. Cela permet de ralentir et réduire la détérioration généralement rapide de la turbine, en limitant les attaques du fluide sur les pales de la turbine.

On notera que seule la partie supérieure 13c de la chambre d'admission peut être associée au reste de la pompe lorsque le carter et la turbine constitue un moteur « brushless ».

Sur la figure 10, on distingue plus en détail le carter 9 constitué d'un corps principal 9a et d'un corps secondaire 9b. Le corps principal 9a est un cylindre allongé, le corps secondaire étant un cylindre dont le diamètre extérieur est inférieur au diamètre extérieur du corps principal. Le corps secondaire 9b est conformé de façon à s'insérer et y être maintenu fixe dans la partie supérieure 13c de la chambre d'admission.

De préférence l'extrémité basse du corps secondaire 9b vient en butée sur la marche 13f, visible sur la figure 8.

La forme intérieure du carter 9 est complémentaire à la forme extérieure de la turbine, avec un entrefer entre eux. L'extrémité supérieure 9d du carter 9 comporte un diamètre intérieur supérieur au diamètre de la partie inférieure 9c ; la partie inférieure logeant la turbine. Le diffuseur 14 et le redresseur 15 viennent loger dans cette partie supérieure 9d du carter 9. La turbine n'occupe donc pas toute la longueur du carter. Le maintien de la turbine dans le carter peut se faire en utilisant des roulements et des joints d'étanchéité.

La turbine selon l'invention permet de communiquer l'énergie cinétique au fluide par sa forme particulière. Elle modifie la vitesse du fluide sans cisaillement et augmente aussi sa pression. Pour se faire, les éléments de sortie de la pompe contribuent à augmenter la pression en présentant un orifice de sortie réduit ainsi que des formes spécifiques.

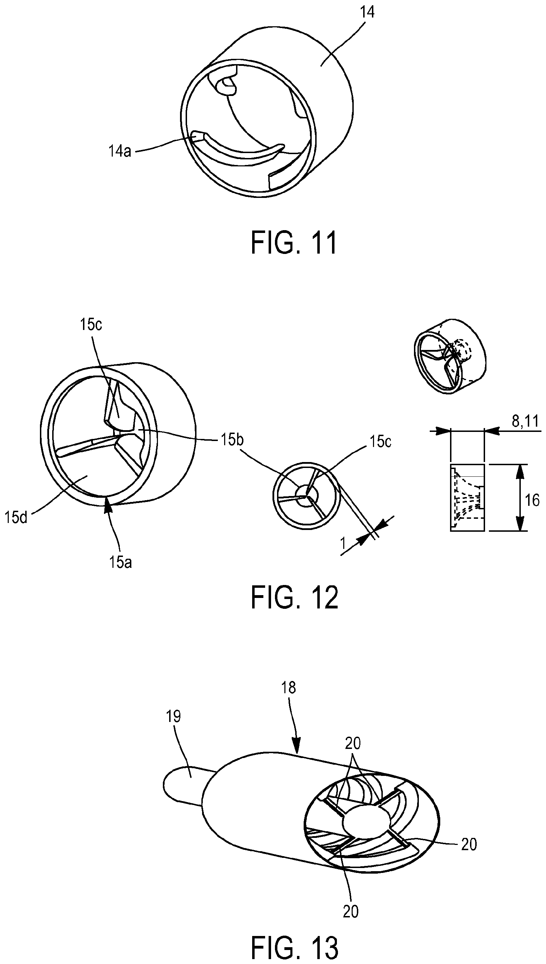

Sur la figure 11, on voit le diffuseur 14 qui est un cylindre destiné à venir coiffer la tête de I turbine. Le diffuseur comporte des pales de guidage orientées 14a dans le sens du refoulement du fluide amené par la turbine. L'orientation des pales de guidage permet de transformer une partie de l'énergie cinétique du fluide en pression qui est une énergie potentielle. L'épaisseur des pales de guidage peut être fixe le long du diffuseur ou bien variable.

Sur la figure 12, on voit un redresseur 15 ayant pour rôle de guider le refoulement du fluide en créant un écoulement laminaire de façon à éliminer toutes turbulences. Il s'agit d'un cylindre ouvert 15a sur sa base pour recevoir le fluide provenant de la turbine via le diffuseur 14. Il comporte un orifice 15b de diamètre inférieur par rapport au diamètre de l'ouverture 15a sur sa base.

On distingue une paroi intérieure 15d de forme conique concave depuis l'ouverture 15a sur sa première moitié puis conique convexe sur sa deuxième moitié vers l'orifice 15b. Le fluide est mis sous pression lorsque poussé vers l'orifice de faible diamètre.

On distingue également trois pales de guidage 15c inscrites dans des plans radiaux qui concourent au centre du redresseur. Chaque pale est une lame de largeur plus épaisse du côté de la paroi que du côté du centre du cylindre. La largeur s'affine donc en s'éloignant de la paroi du cylindre.

Dans la configuration décrite, pour chaque pale de guidage, le profil du côté faisant face à l'axe de rotation du cylindre, est courbé, notamment en arc de cercle, de telle sorte que les pales de guidage se rapprochent les unes des autres au niveau de l'orifice et sont plus éloignées du côté de l'ouverture 15a.

La turbine 1 selon l'invention forme avec le carter 9 et des éléments électroniques et magnétiques, un moteur « brushless » capable de faire circuler le fluide.

Sur la figure 13 est représentée une autre turbine 18 selon l'invention. Cette turbine 18 peut être identique à la turbine 1 décrite ci-dessus, avec ou sans les composants magnétiques (enroulements et aimants permanents et tout autre élément permettant de constituer un moteur). Lorsqu'elle ne comporte pas d'aimants permanents, le corps de la turbine peut être de conception plus légère et plus fine.

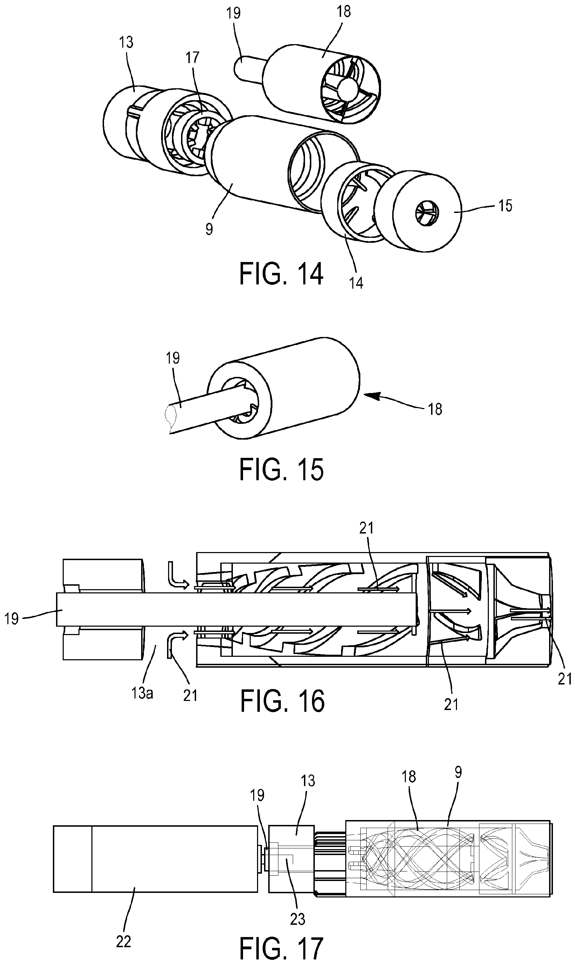

La différence notable avec la turbine 1 est la présence ici d'un arbre de transmission 19 qui est fixé à la turbine 18. Cet arbre de transmission 19 est inséré dans l'axe de la turbine 18 et présente une longueur supérieure de sorte qu'il dépasse la turbine par la partie inférieure (amont) et ne dépasse par l'extrémité supérieure (en aval) de la turbine. L'extrémité supérieure de l'arbre de transmission 19 comporte quatre arêtes ou tiges 20, chacune reliée à l'extrémité d'une pale.. Comme on peut le voir sur la figure 15, on prévoit également que l'arbre de transmission soit fixé au niveau de l'extrémité inférieure (amont) de la turbine 18, directement ou via des arêtes aux extrémités inférieures des pales. De cette manière, l'arbre de transmission est solidement relié à la turbine et la rotation de cet arbre provoquant la rotation de la turbine. Cet arbre 19 peut être l'arbre de transmission d'un moteur externe ou bien une tige indépendante apte à venir en prise avec un moteur.

La figure 14 est une vue éclatée dans laquelle on retrouve les mêmes éléments que su la figure 5 mais avec une turbine 18 à la place de la turbine 1. L'arbre de transmission passe dans l'axe de révolution de l'inducteur 17 et de la chambre d'admission 13. Le carter 9 peut également être différent, plus simplifié, lorsqu'il ne comporte pas d'enroulements magnétiques pour constituer un stator.

Ill est possible d'envisager une turbine 18 et un carter 9 commandé à la fois par un moteur extérieur via l'arbre de transmission 19 et par un moteur asynchrone ou « brushless » constitué par la turbine 18 et le carter 9. A noter que le moteur externe peut également avantageusement être de type « brushless ».

Sur la figure 16, les flèches 21 représentent la circulation du fluide tel que le sang à l'intérieur de la pompe. Le sang arrive radialement à travers les ouvertures 13a de la chambre d'admission, puis est orienté axialement le long de l'arbre de transmission 19 via l'inducteur 17, l'arbre de transmission étant en rotation ainsi que la turbine 18. Le sang est ensuite évacué via le diffuseur 14 et le redresseur 15.

Le flux de sang est ensuite évacué vers l'extérieur en passant par le diffuseur 14 et le redresseur 15 qui par son orifice de sortie crée un flux laminaire de forte pression. La pompe est prévue pour fonctionner en immersion selon une fréquence allant de 500 à 10000 tr/min.

Sur la figure 17 est représenté une pompe selon l'invention intégrant la turbine 18 ainsi qu'un moteur « brushless » externe 22. Ce dernier est fermement connecté à l'arbre de transmission 19 au moyen d'un arbre moteur 23. De préférence cette connexion est amovible de sorte qu'il est possible de remplacer aisément le moteur en cas de défaillance. Il peut s'agir d'une connexion par clip, par vissage ou autre. Cette connexion peut également être la seule liaison entre le moteur et le reste de la pompe.

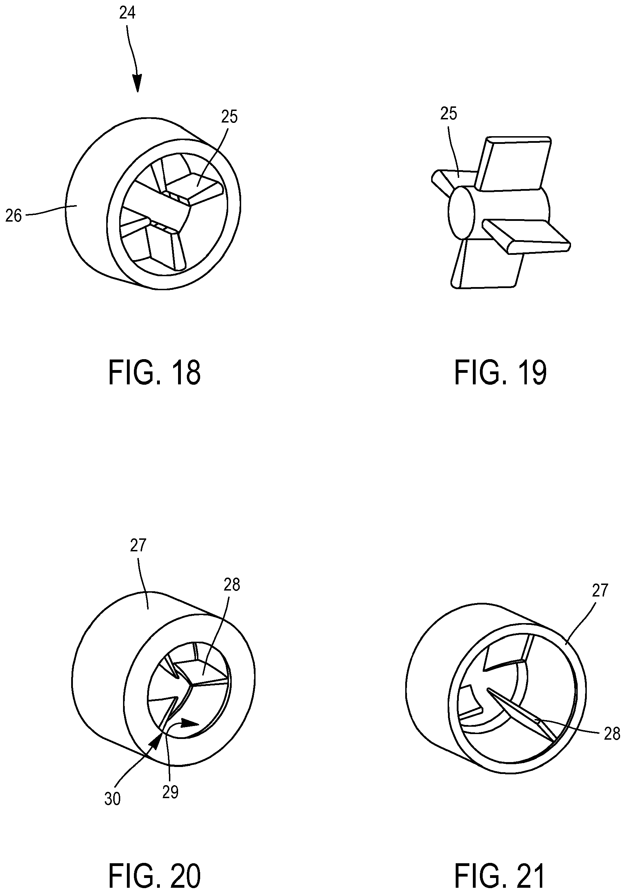

Sur les figures 18 et 19, on distingue une représentation schématique d'un inducteur individuel 24 sur la figure 18 et une représentation schématique uniquement des pales 25 de cet inducteur sur la figure 19. Cet inducteur peut être conçu individuellement. Il sera alors à fixer fermement dans une chambre d'admission. Il peut également être directement réalisé dans une chambre d'admission ou dans un caisson turbine ; dans ce cas ce sont les pales qui sont réalisées sur la paroi interne de la chambre d'admission ou du caisson turbine. La zone centrale de l'inducteur est laissée libre pour le passage du fluide. Sur la figure 19, la zone centrale de l'inducteur représente le flux du fluide.

De façon générale et pour l'ensemble des modes de réalisation, les pales de l'inducteur selon l'invention sont plus épaisses en amont qu'en aval dans le sens de déplacement du fluide. La progression de l'épaisseur peut être linéaire, mais de préférence discontinue : une progression linéaire jusqu'à atteindre une certaine épaisseur, puis l'épaisseur reste constante sur le reste de la longueur de la pale. Par ailleurs, les pales peuvent également être plus épaisses à l'endroit de liaison avec le cylindre 26 qui les porte qu'à l'extrémité centrale. On prévoit également un angle entre la section radiale de chaque pale et le rayon du cylindre 26 portant les pales.

Les figures 20 et 21 illustrent une vue de face et une vue arrière d'un redresseur selon l'invention. Ce redresseur peut être réalisé de façon individuelle puis fixé dans le carter de la pompe ou bien directement réalisé sur la paroi interne du carter, c'est-à-dire une paroi conformée identiquement à la paroi interne du cylindre 27 du redresseur et des pales conçues directement sur cette paroi. Les pales 28 sont plus épaisses en amont qu'en aval dans le sens de circulation du fluide. La progression de l'épaisseur peut être linéaire. Sur la figure 20 est illustré le côté aval du redresseur, c'est-à-dire l'endroit par lequel sort le fluide. Sur la figure 21 est illustré le côté amont du redresseur, l'entrée du fluide. Sur ce dernier côté, les pales 28 laissent un espace centrale plus grand que du côté aval, les pales et la paroi interne 29 du cylindre 27 portant les pales étant conçus de façon à guider le fluide vers l'orifice de sortie 30 qui est plus étroit que l'entrée du redresseur.

Bien sûr, l'invention n'est pas limitée aux exemples qui viennent d'être décrits et de nombreux aménagements peuvent être apportés à ces exemples sans sortir du cadre de l'invention.

La pompe selon l'invention peut facilement être implantée dans un coeur par sa petite dimension du fait de son design particulier permettant une pression élevée tout en conservant la qualité du sang.

La pompe selon l'invention consomme peu du fait qu'elle fonctionne selon le rythme cardiaque physiologique : un flux oscillant.

La pompe selon l'invention fonctionne en propulsant : le rythme est pulsé.

La pompe selon l'invention permet de limiter la surface de contact avec le fluide ainsi que la résistance de la turbine au flux. On limite également les contraintes sur les cellules sanguines (« shear stress »).

Avec une turbine creuse selon l'invention, on gagne considérable en poids, ce qui limite l'énergie nécessaire à son fonctionnement. Cela permet également d'augmenter le volume de passage du flux ainsi que le débit.

Ces caractéristiques avantageuses permettent d'améliorer le confort global d'un patient qui porterait une telle pompe.

La pompe selon l'invention est avantageusement prévue pour fonctionner à la verticale, le rotor étant disposé à la verticale, le fluide pénètre via l'inducteur, traverse le rotor puis ressort par le haut via le diffuseur et le redresseur. La plupart des pompes de l'art antérieur fonctionne à l'horizontale. C'est la capacité d'admission et de refoulement qui permet le fonctionnement à la verticale de la pompe selon l'invention. Une telle pompe, placée dans un ventricule gauche ou droit par exemple, présente l'avantage d'avoir une entrée et une sortie directement dans ce ventricule. Ceci permet d'éviter la présence d'un tube d'entrée et/ou de sortie comme il existe sur d'autres dispositifs de l'art antérieur.

## Revendications

1. Pompe conçue pour la circulation d'un fluide, cette pompe comprenant :
- un carter (9),
- une turbine (1) disposée dans le carter et conçue pour effectuer des mouvements de rotation relativement au carter,
la turbine (1) comprenant :
- un corps (2, 8, 81, 82) conçu pour effectuer des mouvements de rotation,
- une partie centrale creuse (8a, 81a, 82a), traversant le corps de part en part et destinée au passage du fluide à travers la turbine, et
- au moins une pale (4, 5, 6, 7) disposée sur la paroi interne (3) du corps, dans la partie centrale creuse, et conçue pour faire circuler le fluide, une tige (88) disposée dans l'axe centrale de la turbine (8, 81, 82) à une entrée de fluide de façon à réduire les refoulements de fluide, cette tige étant fixée par une extrémité à une chambre d'admission (85) qui est solidaire du carter (9),
**caractérisée en ce que** la pompe comprend en outre:
- un inducteur (17) doté d'aubes de guidage pour rendre l'écoulement du fluide linéaire ; cet inducteur étant disposé en amont de la turbine par rapport au sens de circulation du fluide ;
- un diffuseur (14) doté d'aubes de diffusion pour rendre l'écoulement de fluide linéaire et augmenter la pression du fluide; ce diffuseur étant disposé en aval de la turbine de façon à évacuer le fluide depuis la turbine vers l'extérieur en transformant l'énergie cinétique créée par la turbine en énergie potentielle ; et
- un redresseur (15) doté d'aubes redresseurs et d'un orifice en sortie de diamètre inférieur au diamètre d'entrée du redresseur, les aubes redresseurs dirigeant le fluide, en provenance du diffuseur, vers l'orifice de façon à augmenter la vitesse et donner au fluide un profil prédéterminé en sortie de l'orifice.

2. Pompe selon la revendication 1, **caractérisée en ce que** le carter (9) et la turbine (1) constituent une partie d'un moteur sans balais, ou moteur « brushless », le carter ayant la fonction de stator et la turbine ayant la fonction de rotor.

3. Pompe selon la revendication 2, **caractérisée en ce que** le carter (9) comporte des enroulements statoriques (10) et la turbine comporte des aimants permanents (11).

4. Pompe selon la revendication 1, **caractérisée en ce que** la pompe comprend un moteur extérieur (22) à la turbine et entraînant la tige (88, 19) qui sert d'arbre de transmission (19), cet arbre de transmission étant disposé dans l'axe de rotation de la turbine et solidement connecté par au moins une arrête (20) radiale à la turbine.

5. Pompe selon la revendication 4, **caractérisée en ce que** l'arbre de transmission (19) est connectée à l'extrémité amont et à l'extrémité avale de la pale.

6. Pompe selon la revendication 1, **caractérisée en ce que** la pompe comprend en outre une chambre d'admission (13) pourvue d'ouvertures latérales (13a) pour que le fluide puisse pénétrer radialement puis s'engager dans l'axe vers l'inducteur.

7. Pompe selon la revendication 6, **caractérisée en ce que** la chambre d'admission (13) est de forme cylindrique comportant sur sa partie supérieure en aval desdites ouvertures, un réceptacle pour accueillir l'inducteur (17).

8. Pompe selon l'une quelconque des revendications 1 à 7, **caractérisée en ce que** la pale (4, 5, 6, 7) est de type hélicoïdal.

9. Pompe selon l'une quelconque des revendications 1 à 8, **caractérisée en ce que** la pompe comprend plusieurs pales réparties sur la paroi interne (3) dudit corps.

10. Pompe selon l'une quelconque des revendications 1 à 9, **caractérisée en ce que** la pale est constituée de plusieurs spires à pas d'enroulement croissant et tendant vers l'infini.

11. Pompe selon l'une quelconque des revendications 1 à 10, **caractérisée en ce que** la partie centrale creuse (82a) de la turbine est de forme cylindrique.

12. Pompe selon l'une quelconque des revendications 1 à 10, **caractérisée en ce que** la partie centrale creuse (8a) est de forme évasée, l'extrémité la plus ouverte étant disposée en aval selon le sens de circulation du fluide.

13. Pompe selon l'une quelconque des revendications 1 à 10, **caractérisée en ce que** la partie centrale creuse (81a) de la turbine est de forme oblongue, l'extrémité la plus ouverte étant disposée en aval selon le sens de circulation du fluide.

14. Pompe selon l'une quelconque des revendications 1 à 13, **caractérisée en ce que** ledit corps (2, 8, 81, 82) présente une forme extérieure de type cylindre droit à section circulaire.

15. Pompe selon l'une quelconque des revendications 1 à 14, **caractérisée en ce que** l'épaisseur radiale de la pale est fixe ou variable sur toute la longueur entre l'extrémité amont et l'extrémité aval de cette pale.

16. Pompe selon l'une quelconque des revendications 1 à 10, **caractérisée en ce que** la partie centrale creuse (8a, 81a, 82a) et ladite au moins une pale (4, 5, 6, 7) présentent un profil de type centrifuge du côté amont, de type mixte dans la partie centrale, et de type axial du côté aval.

17. Pompe selon la revendication 16, **caractérisée en ce qu'**une première zone de ladite au moins une pale est dimensionnée pour que le fluide atteigne une vitesse spécifique comprise entre 0 et 1.2 ; cette première zone étant du côté amont de la pale.

18. Pompe selon la revendication 16 ou 17, **caractérisée en ce qu'**une deuxième zone de ladite au moins une pale est dimensionnée pour que le fluide atteigne une vitesse spécifique comprise entre 1 et 2.2 ; cette deuxième zone étant au niveau du milieu de la pale.

19. Pompe selon l'une quelconque des revendications 16 à 18, **caractérisée en ce qu'**une troisième zone de ladite au moins une pale est dimensionnée pour que le fluide puisse atteindre une vitesse spécifique supérieure à 2.2 ; cette deuxième zone étant du côté aval de la pale.

## Patentansprüche

1. Pumpe, die für die Zirkulation eines Fluids konzipiert ist, diese Pumpe umfassend:
- ein Gehäuse (9),
- eine Turbine (1), die in dem Gehäuse angeordnet ist und zum Ausführen von Rotationsbewegungen relativ zu dem Gehäuse konzipiert ist, die Turbine (1) umfassend:
- einen Körper (2, 8, 81, 82), der zum Ausführen von Rotationsbewegungen konzipiert ist,
- einen hohlen Mittelteil (8a, 81a, 82a), der den Körper vollständig durchquert und für den Durchgang des Fluids durch die Turbine bestimmt ist, und
- mindestens eine Laufschaufel (4, 5, 6, 7), die an der Innenwand (3) des Körpers in dem hohlen Mittelteil angeordnet ist und zum Zirkulierenlassen des Fluids konzipiert ist,
einen Stift (88), der in der Mittelachse der Turbine (8, 81, 82) an einem Fluideinlass angeordnet ist, um die Fluidrückströmungen zu reduzieren, wobei dieser Stift durch ein Ende an einer Eintrittskammer (85) befestigt ist, die mit dem Gehäuse (9) fest verbunden ist,
**dadurch gekennzeichnet, dass** die Pumpe ferner umfasst:
- einen Induktor (17), der mit Leitschaufeln zum Linearisieren des Durchflusses des Fluids versehen ist; wobei dieser Induktor relativ zu der Zirkulationsrichtung des Fluids stromaufwärts der Turbine angeordnet ist;
- einen Diffusor (14), der mit Diffusionsschaufeln zum Linearisieren des Durchflusses des Fluids und Erhöhen des Fluiddrucks versehen ist; wobei dieser Diffusor stromabwärts der Turbine angeordnet ist, um das Fluid von der Turbine nach außen abzuleiten, indem die kinetische Energie, die durch die Turbine erzeugt wird, in potenzielle Energie umgewandelt wird; und
- einen Gleichrichter (15), der mit Gleichrichterschaufeln und mit einer Auslassöffnung mit einem Durchmesser versehen ist, der kleiner als der Einlassdurchmesser des Gleichrichters ist, wobei die Gleichrichterschaufeln das Fluid aus dem Diffusor zu der Öffnung lenken, um die Geschwindigkeit zu erhöhen und dem Fluid ein zuvor bestimmtes Profil an dem Auslass der Öffnung zu verleihen.

2. Pumpe nach Anspruch 1, **dadurch gekennzeichnet, dass** das Gehäuse (9) und die Turbine (1) einen Teil eines bürstenlosen Motors oder "Brushless"-Motors ausbilden, wobei das Gehäuse die Funktion eines Stators und die Turbine die Funktion eines Rotors innehat.

3. Pumpe nach Anspruch 2, **dadurch gekennzeichnet, dass** das Gehäuse (9) Statorwicklungen (10) aufweist und die Turbine Permanentmagnete (11) aufweist.

4. Pumpe nach Anspruch 1, **dadurch gekennzeichnet, dass** die Pumpe einen Motor (22) außerhalb der Turbine umfasst und den Stift (88, 19) antreibt, der als Übertragungswelle (19) dient, wobei diese Übertragungswelle in der Rotationsachse der Turbine angeordnet ist und durch mindestens eine Kante (20) radial zu der Turbine fest verbunden ist.

5. Pumpe nach Anspruch 4, **dadurch gekennzeichnet, dass** die Übertragungswelle (19) mit dem stromaufwärtigen Ende und dem stromabwärtigen Ende der Laufschaufel verbunden ist.

6. Pumpe nach Anspruch 1, **dadurch gekennzeichnet, dass** die Pumpe ferner eine Eintrittskammer (13) umfasst, die mit seitlichen Öffnungen (13a) ausgestattet ist, damit das Fluid radial eindringen, dann in der Achse zu dem Induktor gelangen kann.

7. Pumpe nach Anspruch 6, **dadurch gekennzeichnet, dass** die Eintrittskammer (13) eine zylindrische Form vorweist, die in ihrem oberen Teil stromabwärts der Öffnungen einen Behälter zum Aufnehmen des Induktors (17) aufweist.

8. Pumpe nach einem der Ansprüche 1 bis 7, **dadurch gekennzeichnet, dass** die Laufschaufel (4, 5, 6, 7) von spiralförmiger Art ist.

9. Pumpe nach einem der Ansprüche 1 bis 8,
**dadurch gekennzeichnet, dass** die Pumpe mehrere Laufschaufeln umfasst, die über die Innenwand (3) des Körpers verteilt sind.

10. Pumpe nach einem der Ansprüche 1 bis 9,
**dadurch gekennzeichnet, dass** die Laufschaufeln aus mehreren Windungen mit zunehmender Wicklungssteigung besteht und gegen unendlich geht.

11. Pumpe nach einem der Ansprüche 1 bis 10,
**dadurch gekennzeichnet, dass** der hohle Mittelteil (82a) der Turbine eine zylindrische Form vorweist.

12. Pumpe nach einem der Ansprüche 1 bis 10,
**dadurch gekennzeichnet, dass** der hohle Mittelteil (8a) eine aufgeweitete Form vorweist, wobei das am weitesten offene Ende in der Zirkulationsrichtung des Fluids stromabwärts angeordnet ist.

13. Pumpe nach einem der Ansprüche 1 bis 10,
**dadurch gekennzeichnet, dass** der hohle Mittelteil (81a) der Turbine eine längliche Form vorweist, wobei das am weitesten offene Ende in der Zirkulationsrichtung des Fluids stromabwärts angeordnet ist.

14. Pumpe nach einem der Ansprüche 1 bis 13,
**dadurch gekennzeichnet, dass** der Körper (2, 8, 81, 82) eine geradzylinderförmige Außenform mit kreisförmigem Querschnitt darstellt.

15. Pumpe nach einem der Ansprüche 1 bis 14,
**dadurch gekennzeichnet, dass** die radiale Dicke der Laufschaufel über die gesamte Länge zwischen dem stromaufwärtigen Ende und dem stromabwärtigen Ende dieser Laufschaufel fest oder variabel ist.

16. Pumpe nach einem der Ansprüche 1 bis 10,
**dadurch gekennzeichnet, dass** der hohle Mittelteil (8a, 81a, 82a) und die mindestens eine Laufschaufel (4, 5, 6, 7) auf der stromaufwärtigen Seite ein zentrifugenartiges, in dem Mittelteil ein gemischtartiges und auf der stromabwärtigen Seite ein axialartiges Profil darstellen.

17. Pumpe nach Anspruch 16, **dadurch gekennzeichnet, dass** eine erste Zone der mindestens einen Schaufel so bemessen ist, damit das Fluid eine spezifische Geschwindigkeit zwischen 0 und 1,2 erreicht; wobei sich diese erste Zone auf der stromaufwärtigen Seite der Laufschaufel befindet.

18. Pumpe nach Anspruch 16 oder 17, **dadurch gekennzeichnet, dass** eine zweite Zone der mindestens einen Laufschaufel so dimensioniert ist, dass das Fluid eine spezifische Geschwindigkeit zwischen 1 und 2,2 erreicht; wobei diese zweite Zone auf Höhe des Zentrums der Laufschaufel liegt.

19. Pumpe nach einem der Ansprüche 16 bis 18,
**dadurch gekennzeichnet, dass** eine dritte Zone der mindestens einen Schaufel so bemessen ist, dass das Fluid eine spezifische Geschwindigkeit von mehr als 2,2 erreichen kann; wobei sich diese zweite Zone auf der stromabwärtigen Seite der Laufschaufel befindet.

## Claims

1. Pump for circulating a fluid, this pump comprising:
- a casing (9),
- a turbine (1) arranged in the casing and designed to perform rotational movements relative to the casing,
the turbine (1) comprising:
- a body (2, 8, 81, 82) designed to perform rotational movements,
- a hollow central part (8a, 81a, 82a), passing right through the body and intended for the passage of fluid through the turbine, and
- at least one blade (4, 5, 6, 7) arranged on the inner wall (3) of the body, in the hollow central part, and designed to cause the fluid to flow,
- a rod (88) arranged in the central axis of the turbine (8, 81, 82) at a fluid entrance so as to reduce backflows of fluid, the rod (88) being fixed by one end to an inlet chamber (85) which is firmly fixed to the casing (9),
**characterized in that** the pump further comprises :
- an inductor (17) equipped with guide vanes in order to make the flow of the fluid linear; this inductor being arranged upstream of the turbine with respect to the direction of flow of the fluid;
- a diffuser (14) equipped with diffusion vanes in order to make the flow of fluid linear and increase the pressure of the fluid; this diffuser being arranged downstream of the turbine so as to evacuate the fluid outwards from the turbine, converting the kinetic energy created by the turbine into potential energy; and
- a straightener (15) equipped with straightener vanes and an outlet orifice having a diameter less than the inlet diameter of the straightener, the straightener vanes directing the fluid from the diffuser to the orifice so as to increase the speed and give the fluid a predefined profile when leaving the orifice.

2. Pump according to claim 1, **characterized in that** the casing (9) and the turbine (1) constitute a part of a brushless motor, the casing having a stator function and the turbine having a rotor function.

3. Pump according to claim 2, **characterized in that** the casing (9) comprises stator windings (10) and the turbine comprises permanent magnets (11).

4. Pump according to claim 1, **characterized in that** the pump comprises a motor (22) external to the turbine and driving the rod (88, 19) which acts as a transmission shaft (19), the latter being arranged along the axis of rotation of the turbine and solidly connected to the turbine by at least one radial rib (20).

5. Pump according to claim 4, **characterized in that** the transmission shaft (19) is connected at the upstream end and at the downstream end of the blade.

6. Pump according to claim 1, **characterized in that** the pump also comprises an inlet chamber (13) provided with side openings (13a) so that the fluid can enter radially then engage axially towards the inductor.

7. Pump according to claim 6, **characterized in that** the inlet chamber (13) has a cylindrical shape comprising, on its upper part downstream of said openings, a receptacle for housing the inductor (17).

8. Pump according to any one of claims 1 to 7, **characterized in that** the blade (4, 5, 6, 7) is of the helical type.

9. Pump according to any one of claims 1 to 8, **characterized in that** the pump comprises several blades distributed over the inner wall (3) of said body.

10. Pump according to any one of claims 1 to 9, **characterized in that** the blade is constituted by several turns with an increasing winding pitch that tends towards infinity.

11. Pump according to any one of claims 1 to 10, **characterized in that** the hollow central part (82a) of the turbine has a cylindrical shape.

12. Pump according to any one of claims 1 to 10, **characterized in that** the hollow central part (8a) has a flared shape, the more open end being arranged downstream in the direction of flow of the fluid.

13. Pump according to any one of claims 1 to 10, **characterized in that** the hollow central part (81a) of the turbine has an oblong shape, the more open end being arranged downstream in the direction of flow of the fluid.

14. Pump according to any one of claims 1 to 13, **characterized in that** said body (2, 8, 81, 82) has an outer shape of the right cylinder type with a circular cross section.

15. Pump according to any one of claims 1 to 14, **characterized in that** the radial thickness of the blade is fixed or variable over the entire length between the upstream end and the downstream end of this blade.

16. Pump according to any one of claims 1 to 10, **characterized in that** the hollow central part (8a, 81a, 82a) and said at least one blade (4, 5, 6, 7) have a profile of centrifugal type on the upstream side, mixed-flow type in the central part, and axial type on the downstream side.

17. Pump according to claim 16, **characterized in that** a first zone of said at least one blade is dimensioned so that the fluid reaches a specific speed comprised between 0 and 1.2; this first zone being on the upstream side of the blade.

18. Pump according to claim 16 or 17, **characterized in that** a second zone of said at least one blade is dimensioned so that the fluid reaches a specific speed comprised between 1 and 2.2; this second zone being at the level of the middle of the blade.

19. Pump according to any one of claims 16 to 18, **characterized in that** a third zone of said at least one blade is dimensioned so that the fluid can reach a specific speed greater than 2.2; this second zone being on the downstream side of the blade.
